# EUROPEAN PATENT SPECIFICATION

(11) **EP 3 564 786 B1**
(45) Date of publication and mention of the grant of the patent: **15.06.2022**
(21) Application number: 18170445.3
(22) Date of filing: 02.05.2018
(51) Int. Cl.: G06F 3/01, A61B 5/024, A61B 5/0215, A61B 5/026

(54) **AN APPARATUS, METHOD, COMPUTER PROGRAM AND ELECTRONIC DEVICE FOR MONITORING A BIOMETRIC PARAMETER**
VORRICHTUNG, VERFAHREN, COMPUTERPROGRAMM UND ELEKTRONISCHE VORRICHTUNG ZUR ÜBERWACHUNG EINES BIOMETRISCHEN PARAMETERS
APPAREIL, PROCÉDÉ, PROGRAMME INFORMATIQUE ET DISPOSITIF ÉLECTRONIQUE DE SURVEILLANCE D'UN PARAMÈTRE BIOMÉTRIQUE

(43) Date of publication of application: 06.11.2019
(73) Proprietor: Nokia Technologies Oy, 02610 Espoo (FI)
(72) Inventor: O'CONNELL, Diarmuid, Athy, Co. Kildare (IE); BURNS, Ollie, Oldcastle, County Meath (IE)
(74) Representative: Swindell & Pearson Limited

(56) References cited:
- EP-A1- 3 248 539
- WO-A1-2017/014550
- US-A1- 2008 275 322
- US-A1- 2015 190 077

## Description

### TECHNOLOGICAL FIELD

Examples of the present disclosure relate to an apparatus, method, computer program and electronic device for monitoring a biometric parameter. Some relate to an apparatus, method, computer program and electronic device for monitoring a biometric parameter using a wearable device.

### BACKGROUND

Apparatus for monitoring biometric parameters are known. For example optical sensors can be used to obtain information about pule rates, blood oxygen levels, blood glucose levels and other types of parameters.

EP 3 248 539 A1 teaches performing processing on the output of an optical detector to identify motion-related signals, which are larger than blood-flow related changes which are to be monitored, and to remove these signals.

WO 2007/014550 A1 teaches adjusting the brightness of light used to irradiate the human body of the user to compensate for light levels at the sensor units being higher or lower than pre-set references.

US2008/0275322 A1 discloses an optical sensor biosignal measurement apparatus which emits light towards the skin of a user and detects light reflected from the skin.

### BRIEF SUMMARY

The scope of protection sought for various embodiments of the invention is set out by the independent claims, with further optional embodiments as defined by the dependent claims.

According to an example which is not within the scope of the claims there is provided an apparatus comprising means for: receiving an input signal from a sensing array, wherein the sensing array comprises a plurality of light sensors configured to enable at least one biometric parameter of a subject to be monitored; detecting that a change in light detected by at least one sensor within the sensing array is within a threshold range; and providing, in response to detecting that the change is within a threshold range, an output signal enabling the sensing array to be adjusted to compensate for the detected change.

The output signal may cause the sensitivity of at least one of the plurality of light sensors to be adjusted to compensate for the detected change.

The sensing array may comprise a light source and the output signal may cause the intensity of the light source to be adjusted to compensate for the detected change.

The output signal may cause an indication to be provided to a user to instruct the user to adjust the position of the sensing array to compensate for the detected change.

The means may be configured to determine an orientation of the sensing array.

The orientation of the sensing array may be determined by identifying a direction of blood flow.

The output signal may causes a first output if the detected change is within a first threshold range and the second output signal may cause a second output if the detected change is within a second threshold range. The first output may comprise adjustment of the sensors and/or a light source and the second output may comprise an indication to be provided to a user

The plurality of sensors may be configured in a cross shaped arrangement.

The plurality of sensors may be configured in a linear arrangement.

According to an example which is not within the scope of the claims there is provided a method comprising: receiving an input signal from a sensing array, wherein the sensing array comprises a plurality of light sensors configured to enable at least one biometric parameter of a subject to be monitored; detecting that a change in light detected by at least one sensor within the sensing array is within a threshold range; and providing, in response to detecting that the change is within a threshold, an output signal enabling the sensing array to be adjusted to compensate for the detected change.

According to an example which is not within the scope of the claims there is provided a computer program comprising computer program instructions that, when executed by processing circuitry, cause: receiving an input signal from a sensing array, wherein the sensing array comprises a plurality of light sensors configured to enable at least one biometric parameter of a subject to be monitored; detecting that a change in light detected by at least one sensor within the sensing array is within a threshold range; and providing, in response to detecting that the change is within a threshold, an output signal enabling the sensing array to be adjusted to compensate for the detected change.

According to an example which is not within the scope of the claims there is provided a physical entity embodying the computer program as described above.

According to an example which is not within the scope of the claims there is provided an electromagnetic carrier signal carrying the computer program as described above.

According to an example which is not within the scope of the claims there is provided an electronic device comprising an apparatus as described above and a sensing array wherein the sensing array comprises a plurality of light sensors configured to enable at least one biometric parameter of a subject to be monitored.

The electronic device may comprise a transparent encapsulating layer positioned on a first side of the sensing array, so that in use the transparent encapsulating layer is positioned between the sensing array and a subject.

The electronic device may comprise an opaque encapsulation layer positioned, at least partially, around an edge of the transparent encapsulating layer.

The electronic device may also comprise a reflective layer configured to reflect light onto the sensors within the sensing array.

Both the plurality of sensors and the light source may be front mounted on a substrate.

The electronic device may also comprise attachment means configured to enable the electronic device to be attached to a subject.

### BRIEF DESCRIPTION

Some example embodiments will now be described with reference to the accompanying drawings in which:
FIG. 1 shows an example embodiment of an apparatus described herein;
FIG. 2 shows an example embodiment of a sensing array described herein;
FIGS. 3A to 3B show an example embodiments of sensing arrays described herein;
FIG. 4 shows an example embodiment of a method described herein;
Fig. 5 shows another example embodiment of sensing arrays described herein;
FIG. 6 shows an example embodiment of an electronic device described herein;
FIG. 7 shows an example embodiment of an electronic device described herein;
FIG. 8 shows another example embodiment of a method described herein; and
FIGS. 9A to 9F show more example embodiments of sensing arrays described herein

### DETAILED DESCRIPTION

The Figs. illustrate an apparatus 101 configured to receive an input signal from a sensing array 201. The sensing array 201 comprises a plurality of light sensors 203 configured to enable at least one biometric parameter of a subject 301 to be monitored. The apparatus 101 is also configured to detect that a change in light detected by at least one sensor 203 within the sensing array 201 is within a threshold range. This change in the detected light could provide an indication that the sensing array 201 has been moved relative to the subject 301. The apparatus 101 is also configured to provide an output signal in response to detecting that the change is within a threshold. The output signal enables the sensing array 201 to be adjusted to compensate for the detected change. For example the output signal could adjust the sensitivity of one or more sensors 203 within the sensing array 201 and/or could provide an output to a user to instruct them to adjust the position of the sensing array 201.

Examples of the disclosure therefore provide an apparatus 101 for monitoring biometric signals which can take into factors which may affect the sensitivity of the sensors 201. For example the apparatus 101 may be able to adjust the sensing array 201 to take into account small movements of the sensing array 201 and/or dirt or other substances affecting the sensitivity of the sensors 203. In some examples factors such as the skin colour of the subject may affect the amount of light detected. For instance skin with a darker pigmentation may absorb more light than skin with a lighter pigmentation. In some examples the apparatus 101 could be configured to be adjusted for the different skin colours. The apparatus 101 may also be configured to enable a user to be alerted if the sensing array 201 has moved away from the measuring position.

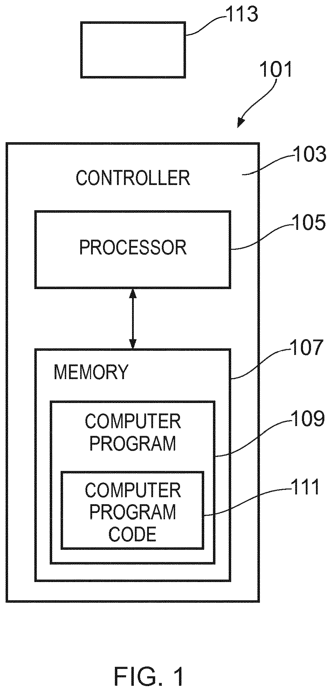

Fig.1 schematically illustrates an apparatus 101 according to examples of the disclosure. In the example of Fig. 1 the apparatus 101 comprises a controller 103. In the example of Fig. 1 the implementation of the controller 103 may be as controller circuitry. In some examples the controller 103 may be implemented in hardware alone, have certain aspects in software including firmware alone or can be a combination of hardware and software (including firmware).

As illustrated in Fig. 1 the controller 103 may be implemented using instructions that enable hardware functionality, for example, by using executable instructions of a computer program 109 in a general-purpose or special-purpose processor 105 that may be stored on a computer readable storage medium (disk, memory etc) to be executed by such a processor 105.

The processor 105 is configured to read from and write to the memory 107. The processor 105 may also comprise an output interface via which data and/or commands are output by the processor 105 and an input interface via which data and/or commands are input to the processor 105.

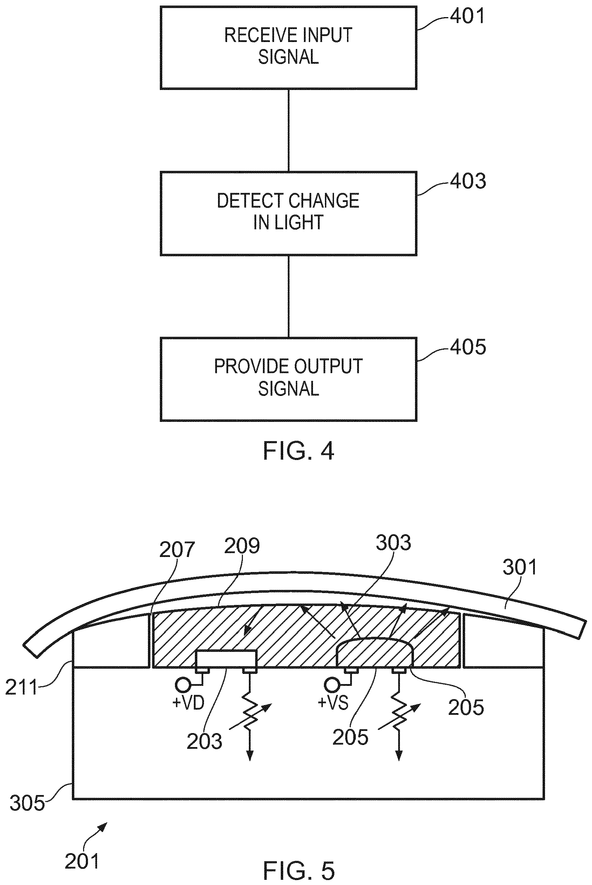

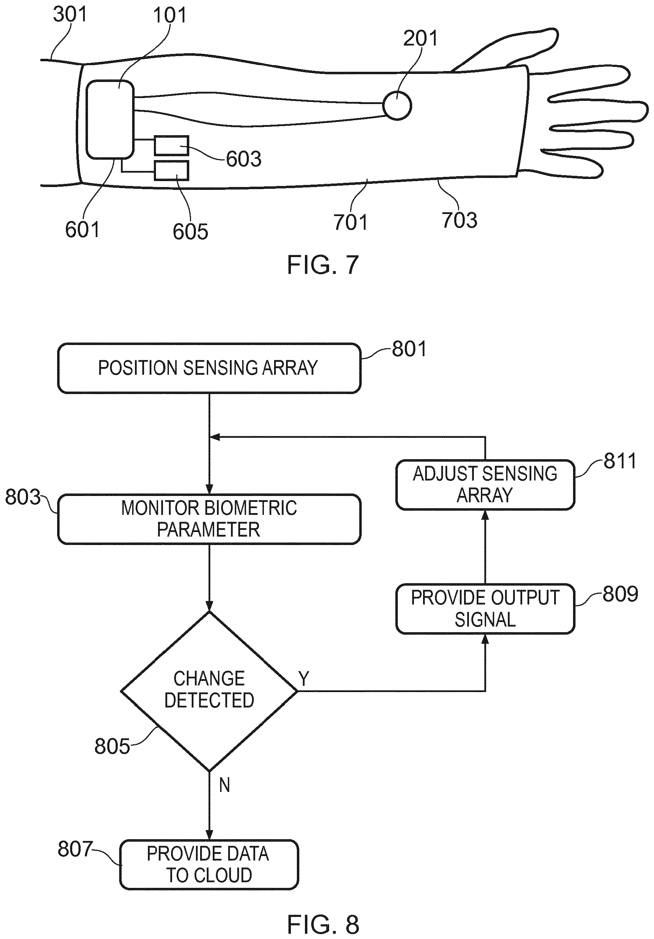

The memory 107 is configured to store a computer program 109 comprising computer program instructions (computer program code 111) that controls the operation of the apparatus 101 when loaded into the processor 105. The computer program instructions, of the computer program 109, provide the logic and routines that enables the apparatus 101 to perform the methods illustrated in Figs. 4 and 8. The processor 105 by reading the memory 107 is able to load and execute the computer program 109.

The apparatus 101 therefore comprises: at least one processor 105; and at least one memory 107 including computer program code 111, the at least one memory 107 and the computer program code 111 configured to, with the at least one processor 105, cause the apparatus 101 at least to perform: receiving an input signal from a sensing array 201, wherein the sensing array 201 comprises a plurality of light sensors 203 configured to enable at least one biometric parameter of a subject 301 to be monitored; detecting that a change in light detected by at least one sensor 203 within the sensing array 201 is within a threshold range; and providing, in response to detecting that the change is within a threshold, an output signal enabling the sensing array 201 to be adjusted to compensate for the detected change.

As illustrated in Fig.1 the computer program 109 may arrive at the apparatus 101 via any suitable delivery mechanism 113. The delivery mechanism 113 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 109. The delivery mechanism may be a signal configured to reliably transfer the computer program 109. The apparatus 101 may propagate or transmit the computer program 109 as a computer data signal. In some examples the computer program 109 may be transmitted to the apparatus 101 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 109 comprises computer program instructions for causing an apparatus 101 to perform at least the following: receiving an input signal from a sensing array 201, wherein the sensing array 201 comprises a plurality of light sensors 203 configured to enable at least one biometric parameter of a subject 301 to be monitored; detecting that a change in light detected by at least one sensor 203 within the sensing array 201 is within a threshold range; and providing, in response to detecting that the change is within a threshold, an output signal enabling the sensing array 201 to be adjusted to compensate for the detected change.

The computer program instructions may be comprised in a computer program 109, a non-transitory computer readable medium, a computer program product, a machine readable medium. In some but not necessarily all examples, the computer program instructions may be distributed over more than one computer program 109.

Although the memory 107 is illustrated as a single component/circuitry it may be implemented as one or more separate components/circuitry some or all of which may be integrated/removable and/or may provide permanent/semi-permanent/ dynamic/cached storage.

Although the processor 105 is illustrated as a single component/circuitry it may be implemented as one or more separate components/circuitry some or all of which may be integrated/removable. The processor 105 may be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

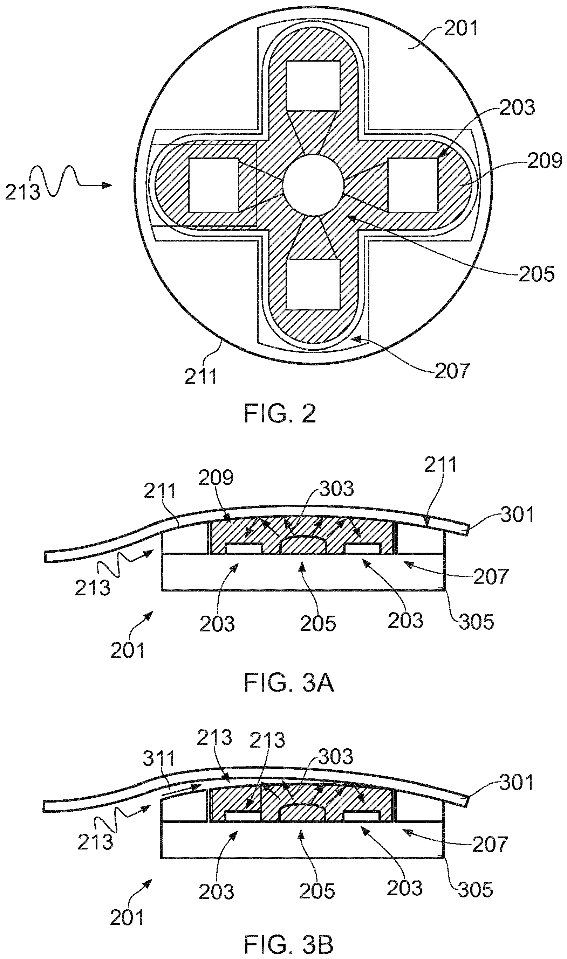

Fig. 2 illustrates an example sensing array 201 which may be used in some examples of the disclosure. The sensing array 201 may be coupled to the apparatus 101 so that the apparatus 101 can receive an input signal from the sensing array 201. In some examples the sensing array 201 may be coupled to the apparatus 101 so that the apparatus 101 can provide an output signal to the sensing array 201.

The sensing array 201 comprises a plurality of sensors 203. In the example of Fig. 2 a light source 205 and a reflector 207 are also provided within the sensing array 201.

The sensors 203 may comprise any means which may be configured to transduce incident light into an electrical signal. The sensors 203 are configured to provide an electrical output signal dependent upon the light detected by the sensors 203. The sensors 203 may be configured to detect light within the visible range of the spectrum and/or within the infrared range of the spectrum. In other examples the sensor 203 may be configured to detect other wavelengths of the spectrum. The sensors 203 could comprise photodiodes or any other suitable sensing means.

In the example of Fig. 2 the sensing array 201 comprises four sensors 203. It is to be appreciated that other numbers of sensors 203 could be provided within the sensing array 201 in other examples of the disclosure.

In the example of Fig. 2 the sensors 201 are configured in a cross shaped arrangement. The cross shaped arrangement comprises two sensors 203 positioned in a vertical axis and two sensors 203 positioned in a horizontal axis. The light source 205 is provided at the intersection of the two axis. It is to be appreciated that other configurations for the sensors 203 could be used in other examples of the disclosure. For example the sensors 203 could be provided within an array comprising a plurality of columns and rows where the rows are provided perpendicular, or substantially perpendicular, to the columns. In some examples the array could be a linear array where a single row of sensors 203 is provided. The linear array could be configured to be aligned with an artery of a subject 301 when the sensing array 201 is in use. In other examples, other arrangements could be used, for example circular or hexagonal arrays or any other suitable geometric arrangement.

The light source 205 may comprise any means which may be configured to provide a light signal. The light signal could be in the visible range or in the infrared range of the spectrum, or in any other suitable range of the spectrum. The light source 205 could comprise a light emitting diode or any other suitable type of light source 205.

The light source 205 is positioned within the sensing array 201 so that, in use, light from the light source 205 can be directed towards the subject 301 and reflected back from the subject 301 to the sensors 203. The amount of light reflected back to the sensors 203 enables a biometric parameter of the subject 301 to be monitored. The biometric parameter may comprise a bio-signal that is generated by the subject's body. The biometric parameter may comprise any time varying signal that is generated by the subject's body. The biometric parameter may comprise an autonomic parameter. The autonomic parameter may be controlled subconsciously by the subject 301. The biometric parameter could comprise heart rate, blood oxygen levels, blood glucose levels or any other suitable parameters. The biometric parameter may provide an indication about the health or other physical condition of the subject.

In the example of Fig. 2 the sensing array 201 also comprises a reflector 207. The reflector 207 comprises a surface which has a high reflectivity. The reflector 207 is positioned surrounding, or at least partially surrounding, the sensors 203 in the sensing array 201 and is configured to direct light that has been reflected from the subject 301 onto the sensors 207. The reflector 207 may therefore increase the efficiency of the sensing array 201.

In the example of Fig. 2 the sensing array 201 also comprises a transparent encapsulating layer 209. The transparent encapsulating layer 209 is indicated by the hashed lines in Fig. 2. The transparent encapsulating layer 209 is positioned on a first side of the sensing array 201, so that in use the transparent encapsulating layer 209 is positioned between the sensing array 201 and a subject 301. In the example of Fig. 2 the transparent encapsulating layer 209 is provided overlaying the sensors 203 within the sensing array 201. In the example of Fig. 2 the transparent encapsulating layer 209 is provided overlaying all of the sensors 203 within the sensing array 201.

The transparent encapsulating layer 209 may be fully transparent, substantially transparent or partially transparent. In some examples the transparent encapsulating layer 209 may be transparent to selected frequencies of light. For example the transparent encapsulating layer 209 may be transparent to light within the infrared range of the spectrum but may bock light from other sections of the spectrum. The wavelengths of light that the transparent encapsulating layer 209 is transparent to may be determined by the wavelengths of light that are to be detected by the sensors 203 within the sensing array 201, the type of biometric parameter that is being monitored and/or any other suitable factor.

The transparent encapsulating layer 209 may comprise any suitable material. In some examples the transparent encapsulating layer 209 may comprise a polymeric material. The material used for the transparent encapsulating layer 209 may be soft so that it may be comfortably pressed against the subject's skin when the sensing array 201 is being used.

In some examples the material used for the transparent encapsulating layer 209 may have a high coefficient of friction so as to restrict movement of the sensing array 201 relative to the subject 301. In some examples ridges or grooves or other rough patterns may be provided on the surface of the transparent encapsulating layer 209 so as to increase the coefficient of friction of the surface. This may reduce the chance of the sensing array 201 being moved during use. The ridges or grooves or other rough patterns may be configured so that they do not interfere with the optical path of light from the light source 205.

In the example of Fig. 2 the sensing array 201 also comprises an opaque encapsulation layer 211. The opaque encapsulation layer 211is positioned, at least partially, around an edge of the transparent encapsulating layer 209.

The opaque encapsulation layer 211 may be fully opaque, substantially opaque or partially opaque. In some examples the opaque encapsulation layer 211 may be opaque to selected frequencies of light. For example the opaque encapsulation layer 211 may be opaque to light within the infrared range of the spectrum but may bock light from other sections of the spectrum. The wavelengths of light that the opaque encapsulation layer 211 is opaque to may be determined by the wavelengths of light that are to be detected by the sensors 203 within the sensing array 201, the type of biometric parameter that is being monitored and/or any other suitable factor.

The opaque encapsulation layer 211may comprise any suitable material. In some examples the opaque encapsulation layer 211may comprise a polymeric material. The material used for the opaque encapsulation layer 211may be soft so that it may be comfortably pressed against the subject's skin when the sensing array 201 is being used.

In some examples the material used for the opaque encapsulation layer 211 may have a high coefficient of friction so as to restrict movement of the sensing array 201 relative to the subject 301. In some examples ridges or grooves or other rough patterns may be provided on the surface of the opaque encapsulation layer 211so as to increase the coefficient of friction of the surface.

The opaque encapsulation layer 211 is configured to provide an optical seal around the edge of the sensing array 201 so as to prevent unwanted light leaking into the sensing array 201. When the sensing array 201 is in use movement of the sensing array 201 or other factors may cause light 213 to leak into the sensing array 201. Fig. 2 shows light 213 leaking into the left hand side of the sensing array 201. This will cause the sensor 203 on the left hand side to detect higher light levels than the other sensors 203 within the sensing array 201. This may be a change in the light levels detected by the left hand sensor 203 compared to the other sensor 203. In examples of the disclosure the apparatus 101 can detect if the change in the light detected by the sensor 203 is above a given threshold. For example, the apparatus 101 may be configured to detect if the sensor 203 has been saturated. If the apparatus 101 detects that the change in the light level is within a given threshold then an output signal enabling the sensing array 201 to be adjusted to compensate for the detected change can be provided by the apparatus 101.

Figs. 3A to 3B show cross sections of part of a sensing array 201 that may be used in some examples of the disclosure. The sensing array 201 could be a sensing array 201 as shown in Fig. 2. Corresponding reference numerals are used for corresponding features.

In the example of Figs. 3A and 3B both the plurality of sensors 203 and the light source 205 are front mounted on a substrate 305. The plurality of sensors 203 and the light source 205 are front mounted on a substrate 305 are provided at the same level upon a flat, or substantially flat, substrate 305. The substrate 305 could be a printed circuit board or any other suitable substrate 305.

In Figs. 3A and 3B the sensing array 201 is shown in use. When the sensing array 201 is in use the sensing array 201 is positioned adjacent to the subject 301 so that light 303 from light source 205 is directed towards the subject 301 and reflected from the subject 205 back into the sensors 203.

When the sensing array 201 is in use the transparent encapsulating layer 209 is provided adjacent to the subject 301. The transparent encapsulating layer 209 may be touching the skin of the subject 301. In the example of Figs. 3A and 3B the transparent encapsulating layer 209 is provided overlaying the sensors 203 and the light source 205. This acts to provide an optical path from the light source 205 to the subject 301 and from the subject 301 to the sensors 203. The optical path enables light 303 from the light source 205 to be directed towards the sensors 203 which are positioned to the sides of the light source 205. This may enable both the light source 205 and the sensors 203 to be mounted on the same side of a substrate 305.

In the example of Fig. 3A the sensing array 201 is positioned in a measuring positioned. The opaque encapsulating layer 211 is acting as an optical seal so that the sensors 203 are only detecting light that has originated form the light source 205 and been reflected by the subject 301. In the example of Fig. 3A there is no gap between the opaque encapsulating layer 211 and the skin of the subject 301.

In the example of Fig. 3B the sensing array 201 has been moved so that a gap 311 is provided between the opaque encapsulating layer 211 and the skin of the subject 301. This gap 311 allows light 213 to leak into the sensing array 201. In the example of Fig. 3B this will increase the light detected by the left hand sensor 203. In examples of the disclosure the apparatus 101 may be configured to detect this movement of the sensing array 201 by detecting that the light detected by sensor 203 on the left hand side has changed and that this change is within a predetermined threshold. The apparatus 101 may detect that the sensor 203 on the left hand side is now saturated.

Fig. 4 illustrates an example method that may be used in some examples of the disclosure. The method may be implemented using an apparatus 101 as shown in Fig. 1. The apparatus 101 could be coupled to sensing arrays 201 such as those shown in Figs. 2 to 3B.

The method comprises, at block 401, receiving an input signal from a sensing array 201. The sensing array 201 comprises a plurality of light sensors 203 configured to enable at least one biometric parameter of a subject 301 to be monitored.

At block 403 the method comprises detecting that a change in light detected by at least one sensor 203 within the sensing array 201 is within a threshold range. The change in the light that is detected could be a change compared to the light that has been detected by the sensor 203 at an earlier point in time. In some examples the change could be a change compared to the other sensors 203 within the sensing array 201.

The change that is detected could be an increase in the amount of light detected. For example, if the sensing array 201 is moved so that the optical seal is broken, this will increase the light detected by one or more sensors. The optical seal could be broken if the opaque encapsulating layer 211 is moved away from the surface of the subject 301. In other examples the change that is detected could be a decrease in the amount of light detected. For example, dirt, grease or other contaminants could build up on the surface of the sensing array 201 and decrease the amount of light detected by the sensors 203.

The apparatus 101 may be configured to detect when the change is within a threshold range. This may enable the apparatus 101 to distinguish between changes in the light level which are expected during operation of the sensing array 201 and changes which are caused by other factors such as movement of the sensing array 201 or the build-up of contaminants. The threshold range in which a change causes an output signal to be provided in accordance with this disclosure may be larger, or substantially larger, than the changes expected during normal use of the sensing array 201. For example, changes caused by a subject's pulse may be outside of the threshold range, but changes which cause saturation of the sensors 203 would be within the threshold.

At block 405 the method comprises providing an output signal enabling the sensing array 201 to be adjusted to compensate for the detected change. The output signal is provided in response to the detecting that the change is within the threshold range.

In some examples the output signal may be provided to the sensing array 201 to enable adjustment of the sensing array 201. This may enable automatic adjustment of the sensing array 201. For instance in some examples the output signal could be provided to the sensing array 201 to adjust the sensitivity of one or more of the plurality of light sensors 203 so as to compensate for the change. The sensitivity of the light sensors 203 could be decreased if the detected change is an increase in the mount of light detected. The sensitivity of the light sensors 203 could be increased if the detected change is a decrease in the amount of light detected. In some examples only the sensors 203 which have been affected by the change may be adjusted.

In some examples the output signal could be provided to the sensing array 201 to adjust the intensity of the light source 205 so as to compensate for the detected change. The intensity of the light source 205 could be decreased if the detected change is an increase in the mount of light detected. The intensity of the light source 205 could be increased if the detected change is a decrease in the amount of light detected. In some examples both the intensity of the light source 205 and the sensitivity of the light sensors 203 could be adjusted.

In some examples the output signal could cause an indication to be provided to a user to instruct a user to adjust the position of the sensing array 201 to compensate for the detected change. In such examples the output signal could be provided to an output device such as a display, audio output device, haptic feedback device or any other suitable type of output device. In examples of the disclosure the output that is provided could provide an indication of how the sensing array 201 should be adjusted. For example, the apparatus 101 may determine which of the sensors 203 within the sensing array 203 have been affected and may provide an indication to a user of how to adjust these sensors 203.

In some examples the apparatus 101 may also be configured to determine an orientation of the sensing array 201. The orientation of the sensing array could then be used to help to identify when a change in the light levels is above a threshold level and/or how such a change should be compensated for. Any suitable means could be sued to determine the orientation of the sensing array 201. In some examples the direction of pulse flow could be detected based on the timing of light pulses detected by sensors within the sensing array 201. This information may be used to determine how the sensing array 201 is oriented and can be used to determined how the sensing array 201 should be adjusted if a change in position has been detected. For example, where the sensing array 201 is provided within a wearable device such as a sleeve, determining the orientation of the sensing array 201 may help to identify which of the sensors 203 are closest to the edge of the sleeve and are most likely to be affected by the leakage of light.

Also in some examples of the disclosure the apparatus 101 could be configured to determine a pulse wave velocity. In such examples the sensing array 201 could be configured so that the sensors 203 can be positioned along a radial artery, or other suitable part of the subject. The apparatus 101 could be configured to determine the timings of fluctuations in the light levels detected by the sensors 203 within the sensing array 201 and may be configured to enable this timing information to be used to determine the pulse wave velocity or any other suitable biometric parameter.

In some examples the apparatus 101 may be configured to detect different threshold ranges for the change in light detected and may provide different output signals corresponding to the different ranges. For example a first output signal is provided if the detected change is within a first threshold range and a second output signal is provided if the detected change is within a second threshold range. The first output signal may cause a first output to be provided while the second output signal may cause a second, different output to be provided.

In some examples the first threshold range could be an increase and a second threshold range could be a decrease. In such examples the first output signal could cause the sensitivity of the sensing array 201 to be decreased and the second output signal could cause the sensitivity of the sensing array 201 to be increased. In some examples the first output signals could provide instructions to a user to move the sensing array 201to compensate for an increase in light and could instruct the user to clean the contaminants off the sensing array 201 to compensate for a decrease in light.

In some examples the first threshold range could be an increase of a first value and the second threshold range could be an increase of a second values. In such examples the first output signal could cause the adjustment of the sensors 203 and/or the light source 205. This could enable an automatic adjustment of the sensing array 201 where a small amount of light has leaked in. The second output signal could cause an indication to be provided to a user to instruct the user to reposition the sensing array 201. This could enable manual correction of the sensing array 201 where a larger amount of light has been leaked in.

Fig. 5 illustrates cross sections of part of a sensing array 201 that can be automatically adjusted according to examples of the disclosure. The sensing array 201 could be a sensing array 201 as shown in Figs. 2 and 3A to 3B. Corresponding reference numerals are used for corresponding features. In the example of Fig. 5 only one sensor 203 is shown, however it is to be appreciated that any number of sensors 203 could be used in examples of the disclosure.

In the example of Fig. 5 both the sensor 203 and the light source 205 can be adjusted in response to a signal from the apparatus 101. The sensitivity of the sensor 203 may be adjusted by controlling the voltage V_{D} of the signal provided to the sensor 203. The intensity of the light source 205 can be adjusted by adjusting the voltage Vₛ of the signal provided to the light source 205. Other means for automatically adjusting the sensitivity of the sensing array 201 could be used in other examples of the disclosure.

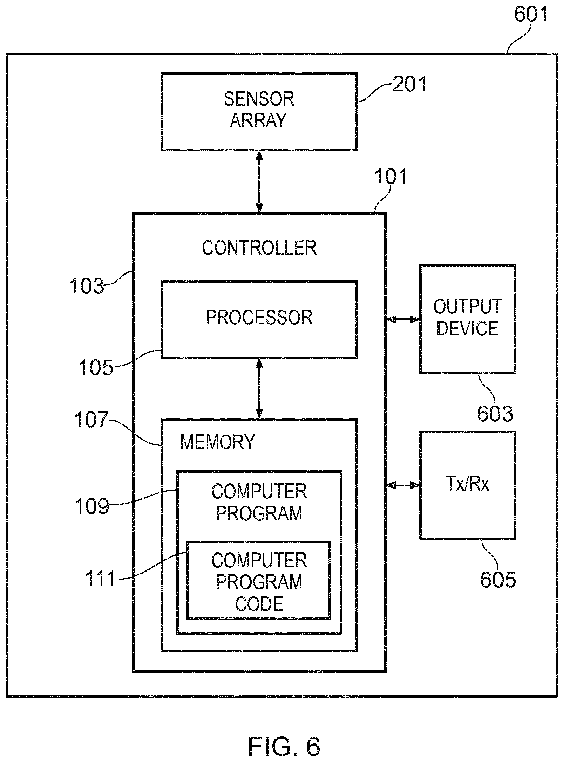

Fig. 6 schematically illustrates an example electronic device 601 which may be provided in some examples of the disclosure. The electronic device 601 could comprise an apparatus 101, at least one sensing array 201, an output device 603 and at least one transceiver 605. The apparatus 101 may be as shown in Fig. 1.

The sensing array 201 could comprise a plurality of light sensors 203 configured to enable at least one biometric parameter of a subject 301 to be monitored. In some examples the sensing array 201 may comprise other types of sensor in addition to the plurality of light sensors 203. For example the sensing array 201 could also comprise temperature sensors, pressure sensors, or any other suitable types of sensors.

The sensing array 201 is coupled to the apparatus 101 so that input signals from the sensing array 201 can be received by the apparatus 101 and output signals from the apparatus 101 can be provided to the sensing array 201. This may enable the apparatus 101 to detect changes in the light levels detected by the sensing array 201 and control the sensing array 201 in response to a detected change.

In the example of Fig. 6 the electronic device 601 also comprises an output device 603. The output device 603 may comprise any means which enables an output to be provided to a user. The user could be the subject 301 who is having the biometric parameters monitored. In some examples the user could be a different person, for example the user could be a medical professional operating the electronic device 601 on behalf of the subject 301.

In some examples the output device 603 may comprise an audio output device such as a loudspeaker. This may enable audible alerts and/or instructions to be provided to a user. In some examples the output device 603 could comprise a display which may enable visual instructions to be provided to the user.

The output device 603 is coupled to the apparatus 101 so that output signals from the apparatus 101 can be provided to the output device 603. This may enable the apparatus 101 to control the output device 603 in response to a detected change from the sensing array 201.

In the example of Fig. 6 the output device 601 is provided as part of the electronic device 601. In other examples the output device 603 could be provide separate to the electronic device 601 and output signals could be transmitted form the electronic device 601 to the output device 603.

In the example of Fig. 6 the electronic device 60 also comprises at least one transceiver 605. The at least one transceiver 605 may comprise any suitable means for receiving and/or transmitting information. The at least one transceiver 605 may comprise one or more transmitters and/or receivers. The at least one transceiver 605may enable a wireless connection between the electronic device 601 and another entity. The wireless connection could be a wireless connection such as a cellular connection, a WiFi connection, a Bluetooth connection or any other suitable type connection.

Fig. 7 illustrates an example electronic device 601 in use. In the example of Fig. 7 the electronic device 601 is a wearable electronic device 601. In the example of Fig. 7 the electronic device 601 comprises a sensing array 201 and an apparatus 101. The electronic device 601 also comprises an output device 603 and/or one or more transceivers 605.

The wearable electronic device 601 comprises attachment means 701 which enables the wearable electronic device 601 to be attached to the subject 301. In the example of Fig. 7 the attachment means 701 comprises a sleeve which can be worn on the user's arm 703. In other examples of the disclosure the attachment means 701 could comprise a strap which could be wrapped around part of the subject 301. For example the strap could be wrapped around the subject's torso, arm, leg or other suitable part of their body.

The sensing array 201 is positioned within the attachment means 701 so that when the attachment means 701 is attached to a subject 301 the sensing array 201 is positioned adjacent to the subject 301. The sensing array 201 is positioned within the attachment means 701 so that when the attachment means 701 is attached to a subject the transparent encapsulating layer 209 of the sensing array 201 is in contact with the skin of the subject 301.

In the example of Fig. 7 only one sensing array 201 is provided within the wearable electronic device 601. In other examples a plurality of different sensing arrays 201 could be provided at different positions within the wearable electronic device 601. This may enable the biometric parameter to be monitored at different positions of the subject's body. In some examples the different sensing arrays 201 could be used to monitor different biometric parameters.

In some but not necessarily all examples, the apparatus 101 is configured to communicate data from the apparatus 101 with or without local storage of the data in a memory 107 at the apparatus 101 and with or without local processing of the data by circuitry or processors at the apparatus 101.

The data may, for example, be measurement data from the sensing array 201 or data produced by the processing of measurement data from sensing array 201. The data produced by processing the measurement data could be a pulse rate, a blood oxygen level, a blood glucose level or any other suitable data.

The data may be stored in processed or unprocessed format remotely at one or more devices. The data may be stored in the Cloud.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The apparatus may comprise a communications interface such as, for example, a radio transceiver for communication of data.

The apparatus 101 may be part of the Internet of Things forming part of a larger, distributed network.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train a machine learning network or may be used as a query input to a machine learning network, which provides a response. The machine learning network may for example use linear regression, logistic regression, vector support machines or an acyclic machine learning network such as a single or multi hidden layer neural network.

The processing of the data, whether local or remote, may produce an output. The output may be communicated to the apparatus 101 where it may produce an output sensible to the subject such as an audio output, visual output or haptic output.

Fig. 8 illustrates another example method. The method may be implemented using an electronic device 601 as shown in Figs. 6 or 7 or by any other suitable type of electronic device 601.

The method comprises, at block 801, positioning the sensing array 201 at a measuring position. In some examples this block could comprise attachment means 701 being attached to a subject 301, for example a sleeve, such as the sleeve shown in Fig. 7 could be placed on a subject's arm.

At block 803 the biometric parameter if the subject is monitored. The output signal of the sensing array 201 is used to monitor the biometric parameter.

At block 805 it is determined whether or not a threshold change has been detected in the light detected by one or more of the sensors 203 within the sensing array 201. If no change is detected, or if any detected change is outside of a threshold, then at block 807 the data obtained from the sensing array 201 is provided to the cloud. In some examples the data may be processed before it is sent to the cloud. In other examples the data may be sent to the cloud to enable the data to be processed.

If a change is detected, and the change is within a threshold range, then at block 809 an output signal is provided by the apparatus 101. At block 811 the output signal causes adjustment of the sensing array 201. In some examples the output signal may cause automatic adjustment of the sensing array 201 by adjusting the sensitivity of the sensors 203 and/or by adjusting the intensity of a light source 205. In some examples the output signal may enable manual adjustment of the sensing array 201 by instructing a user to move the sensing array 201.

Once the sensing array 201 has been adjusted the method returned to block 803 and the monitoring of the biometric parameter is continued.

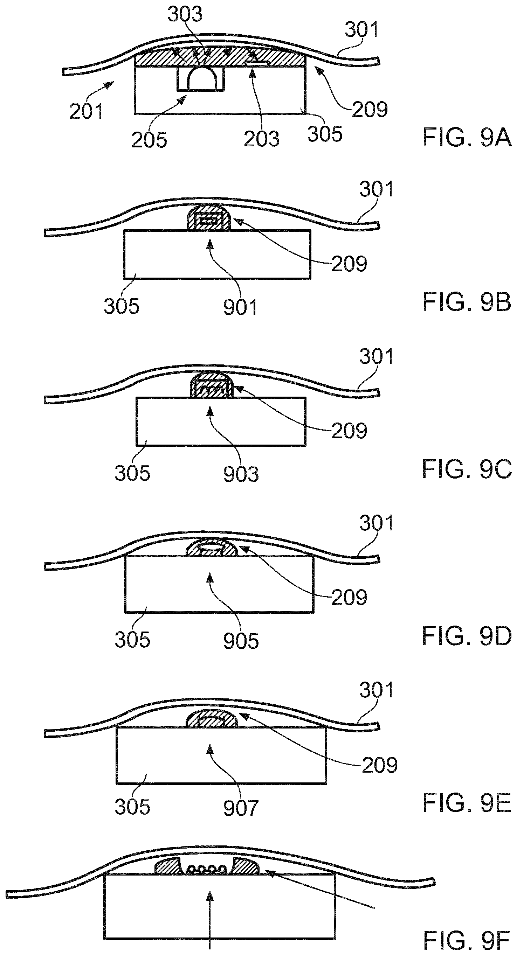

Figs. 9A to 9H show different types of sensors, additional sensors and additional output devices that may be provided within a sensing array 201 in some examples of the disclosure.

In the example of Fig. 9A the sensor 203 is a light sensor 203 which is provided adjacent to a light source 205. In the example of Fig. 9A the light source 205 is mounted on a different surface to the sensor 203. In the example of Fig. 9A the sensor 203 may be front mounted on the substrate 305 while the light source 205 may be rear mounted on the same substrate 305. This may increase the optical path length between the light source 205 and the subject 301 and may enable a greater proportion of the light 303 from the light source 205 to be reflected back to the sensors 203.

In the example of Fig. 9B a haptic output device 901 is provided. The haptic output device 901 may comprise any means which provides a movement that can be perceived by the subject's sense of touch. In the example of Fig. 9B the haptic output device 901 comprises a motor which is configured to move such that a subject 301 can feel the movement of the motor or one or more components coupled to the motor. Other types of haptic output device 901 could be used in other examples of the disclosure. For example the haptic output device 901 could comprise an electro active polymer or any other suitable means which may be configured to change shape in response to an input signal.

The haptic output device 901 is positioned within a sensing array 201 so that when the sensing array 201 is in use the haptic output device 901 is positioned adjacent to the subject's skin so that the subject 901 can feel the movement of the haptic output device 901.

In the example of Fig. 9B an encapsulating layer 209 is provided over the haptic output device 901. The encapsulating layer may 209 be a soft layer so that movement of the haptic output device 901 can be transferred from the haptic output device 901 to the subject 301 via the encapsulating layer 209. In the example of Fig. 9B the encapsulating layer 209 is a transparent encapsulating layer 209. In other examples the encapsulating layer could be an opaque encapsulating layer 211.

In the example of Fig. 9C a thermal output device 903 is provided. The thermal output device 903 may comprise any means which provides a change in temperature that can be perceived by the subject 301. In the example of Fig. 9C the thermal output device 903 comprises a heater which is configured to provide an increase in temperature that the subject 301 can feel. Other types of thermal output device 903 could be used in other examples of the disclosure.

The thermal output device 903 is positioned within a sensing array 201 so that when the sensing array 201 is in use the thermal output device 903 is positioned adjacent to the subject's skin so that the subject 901 can feel the change in temperature.

In the example of Fig. 9C an encapsulating layer 209 is provided over the thermal output device 903. The encapsulating layer may 209 be a thermally conductive layer so that heat from the thermal output device 903 can be transferred from the thermal output device 903 to the subject 301 via the encapsulating layer 209. In the example of Fig. 9C the encapsulating layer 209 is a transparent encapsulating layer 209. In other examples the encapsulating layer 209 could be an opaque encapsulating layer 211.

The output devices 901, 903 shown in Figs. 9B and 9C could be used to provide an indication to the subject that an error has occurred in the sensing array. For example the apparatus 101 could control the output devices 901, 903 to provide an output to the subject 301 if a detected change is within a threshold. This could trigger the user to move the sensor array 201 or otherwise adjust the sensing array 201.

Fig. 9D illustrates another type of sensor that could be provided within the sensing array 201. In the example of Fig. 9D the sensor comprises an electrode 905. The electrode 905 may enable an electrical signal to be provided to the subject 301 to enable electrical properties of the subject 301 to be measured. For example the electrode 905 could be an ECG (electrocardiogram) electrode or any other suitable type of electrode 905.

In some examples the electrode 905 could enable an electrical signal to be provided to the subject 301 to stimulate for the subject 301. In some examples this stimulation could be used to provide haptic feedback to the subject 301.

In the example of Fig. 9D an encapsulating layer 209 is provided over the electrode 905. The encapsulating layer may 209 be an electrically conductive layer so that a signal from the electrode 905 can be transferred from the electrode 905 to the subject 301 via the encapsulating layer 209. In the example of Fig. 9D the encapsulating layer 209 is a transparent encapsulating layer 209. In other examples the encapsulating layer could be an opaque encapsulating layer 211.

Fig. 9E illustrates another type of sensor that could be provided within the sensing array 201. In the example of Fig. 9E the sensor comprises an ultrasound sensor 907. An ultrasound source may also be provided. The ultrasound sensor 907 and ultra sound source may enable an ultrasound signal to be provided to the subject 301 to enable biometric parameters of the subject 301 to be measured.

In the example of Fig. 9E an encapsulating layer 209 is provided over the ultrasound sensor 907. The encapsulating layer may 209 be selected to provide impedance matching between the subject 301 and the encapsulating layer 209 so as to reduce reflections of the ultrasound signal at the interface between the subject 301 and the encapsulating layer 209. In the example of Fig. 9E the encapsulating layer 209 is a transparent encapsulating layer 209. In other examples the encapsulating layer could be an opaque encapsulating layer 211.

Fig. 9F illustrates another type of sensor that could be provided within the sensing array 201. In the example of Fig. 9F the sensor comprises a chemical sensor 909. The chemical sensor 909 may comprise any means which detects the presence of particular chemical or analytes and provides an electrical output signal in response. The chemical sensor 909 may be configured to detect water or other chemicals from the subject 301.

In the example of Fig. 9F an encapsulating layer 209 is provided around the chemical sensor 907. A gap is provided in the encapsulating layer 209 so that chemicals can reach the chemical sensor 909. In other examples the encapsulating layer 209 may be porous to enable the chemical that is to be detected to be transferred through the encapsulating layer 209. In the example of Fig. 9F the encapsulating layer 209 is a transparent encapsulating layer 209. In other examples the encapsulating layer could be an opaque encapsulating layer 211.

Examples of the disclosure therefore provide apparatus 101 and methods which enable sensing of biometric parameters. In examples of the disclosure the sensing arrays 201 can be adjusted to take into account errors which may occur due to movement of the sensing arrays 201, contaminants blocking the sensing arrays 201or any other suitable factors. This provides for a reliable measurement of the biometric parameters.

The term coupled has been used to mean operationally coupled. Any number or combination of intervening elements can exist between coupled components, including no intervening elements.

The above described examples find application as enabling components of:
automotive systems; telecommunication systems; electronic systems including consumer electronic products; distributed computing systems; media systems for generating or rendering media content including audio, visual and audio visual content and mixed, mediated, virtual and/or augmented reality; personal systems including personal health systems or personal fitness systems; navigation systems; user interfaces also known as human machine interfaces; networks including cellular, non-cellular, and optical networks; ad-hoc networks; the internet; the internet of things; virtualized networks; and related software and services.

The blocks illustrated in Figs. 4 and 8 may represent steps in a method and/or sections of code in the computer program 111. The illustration of a particular order to the blocks does not necessarily imply that there is a required or preferred order for the blocks and the order and arrangement of the block may be varied. Furthermore, it may be possible for some blocks to be omitted.

## Claims

1. An electronic device (601) for monitoring at least one biometric parameter comprising:
a light source (205) for providing a light signal in a direction towards a subject (301);
a sensing array (201) for detecting light reflected by the subject, wherein the sensing array comprises a plurality of light sensors (203) configured to enable at least one biometric parameter of the subject to be monitored;
means for detecting that a change in light level detected by at least one sensor within the sensing array is within a threshold range; and **characterised by** comprising:
means for providing, in response to detecting that the change is within a threshold range, an output signal enabling the sensing array (201) to be adjusted, wherein the output signal causes a sensitivity of at least one of the plurality of light sensors (203) to be adjusted to compensate for the detected change.

2. An electronic device (601) as claimed in claim 1 wherein the output signal causes the intensity of the light source to be adjusted to compensate for the detected change.

3. An electronic device (601) as claimed in any preceding claim comprising means configured to detect that a change in light detected by at least one sensor within the sensing array is within a second, different threshold range and to cause, in response to detecting that the change is within the second threshold range, an indication to be provided to a user to instruct the user to adjust the position of the sensing array to compensate for the detected change.

4. An electronic device (601) as claimed in claim 3 comprising means configured to determine an orientation of the sensing array to enable determination of how the position of the sensing array should be adjusted if the detected change is within the second threshold range.

5. An electronic device (601) as claimed in claim 4 wherein the orientation of the sensing array is determined by identifying a direction of blood flow.

6. An electronic device (601) as claimed in any preceding claim wherein the plurality of sensors are configured in a cross shaped arrangement or in a linear arrangement.

7. An electronic device (601) as claimed in any previous claim, wherein the sensitivity of the at least one sensor is adjusted by controlling the voltage of the signal provided to the at least one sensor.

8. An electronic device (601) as claimed in any of claims 1 to 7 comprising a transparent encapsulating layer (209) positioned on a first side of the sensing array, so that in use the transparent encapsulating layer is positioned between the sensing array and a subject.

9. An electronic device as claimed in 8 comprising an opaque encapsulation layer (211) positioned, at least partially, around an edge of the transparent encapsulating layer.

10. A method for monitoring at least one biometric parameter comprising:
providing a light signal in a direction towards a subject (301);
detecting light reflected by the subject at a sensing array (201), wherein the sensing array comprises a plurality of light sensors (203) configured to enable at least one biometric parameter of the subject to be monitored;
detecting that a change in light level detected by at least one sensor within the sensing array is within a threshold range; and **characterised by** comprising:
providing, in response to detecting that the change is within a threshold range, an output signal enabling the sensing array (201) to be adjusted, wherein the output signal causes a sensitivity of at least one of the plurality of light sensors (203) to be adjusted to compensate for the detected change.

11. A method as claimed in claim 10 wherein the output signal causes the intensity of the light source to be adjusted to compensate for the detected change.

12. A method as claimed in claim 10 or claim 11 comprising detecting that a change in light detected by at least one sensor within the sensing array is within a second, different threshold range and causing, in response to detecting that the change is within the second threshold range, an indication to be provided to a user to instruct the user to adjust the position of the sensing array to compensate for the detected change.

13. A method as claimed in claim 12 comprising determining an orientation of the sensing array to enable determination of how the position of the sensing array should be adjusted if the detected change is within the second threshold range.

14. A method as claimed in claim 13 wherein the orientation of the sensing array is determined by identifying a direction of blood flow.

15. A computer program (109) comprising computer program instructions that, when executed by processing circuitry (105) of an electronic device according to any one of claims 1 to 9 , cause:
providing a light signal in a direction towards the subject (301);
detecting light reflected by the subject at the sensing array (201), wherein the sensing array comprises a plurality of light sensors (203) configured to enable at least one biometric parameter of the subject to be monitored;
detecting that a change in light level detected by at least one sensor within the sensing array is within a threshold range; and **characterised by**
providing, in response to detecting that the change is within a threshold range, an output signal enabling the sensing array (201) to be adjusted, wherein the output signal causes a sensitivity of at least one of the plurality of light sensors to be adjusted to compensate for the detected change.

## Patentansprüche

1. Elektronische Vorrichtung (601) zum Überwachen von mindestens einem biometrischen Parameter, die Folgendes umfasst:
eine Lichtquelle (205) zum Bereitstellen eines Lichtsignals in einer Richtung zu einem Patienten (301);
ein Erfassungsarray (201) zum Detektieren von Licht, das vom Patienten reflektiert wird, wobei das Erfassungsarray eine Vielzahl von Lichtsensoren (203) umfasst, die dazu ausgelegt sind, es zu ermöglichen, dass mindestens ein biometrischer Parameter des Patienten überwacht wird;
Mittel zum Detektieren, dass eine Änderung einer Lichtstärke, die von mindestens einem Sensor im Erfassungsarray detektiert wird, innerhalb eines Schwellwertbereichs liegt; und **dadurch gekennzeichnet, dass** sie Folgendes umfasst:
Mittel zum Bereitstellen eines Ausgangssignals zum Ermöglichen des Anpassens des Erfassungsarrays (201) in Reaktion auf das Detektieren, dass die Änderung innerhalb eines Schwellwertbereichs liegt, wobei das Ausgangssignal bewirkt, dass eine Empfindlichkeit von mindestens einem der Vielzahl von Lichtsensoren (203) angepasst wird, um die detektierte Änderung auszugleichen.

2. Elektronische Vorrichtung (601) nach Anspruch 1, wobei das Ausgangssignal bewirkt, dass die Intensität der Lichtquelle angepasst wird, um die detektierte Änderung auszugleichen.

3. Elektronische Vorrichtung (601) nach einem der vorhergehenden Ansprüche, die Mittel umfasst, die dazu ausgelegt sind zu detektieren, dass eine Änderung des Lichts, die von mindestens einem Sensor im Erfassungsarray detektiert wird, innerhalb eines zweiten, anderen Schwellwertbereichs liegt, und in Reaktion auf das Detektieren, dass die Änderung innerhalb des zweiten Schwellwertbereichs liegt, das Bereitstellen einer Anzeige für einen Benutzer zu bewirken, um den Benutzer anzuweisen, die Position des Erfassungsarrays anzupassen, um die detektierte Änderung auszugleichen.

4. Elektronische Vorrichtung (601) nach Anspruch 3, die Mittel umfasst, die dazu ausgelegt sind, eine Ausrichtung des Erfassungsarrays zu bestimmen, um es zu ermöglichen zu bestimmen, wie die Position des Erfassungsarrays angepasst werden sollte, wenn die detektierte Änderung innerhalb des zweiten Schwellwertbereichs liegt.

5. Elektronische Vorrichtung (601) nach Anspruch 4, wobei die Ausrichtung des Erfassungsarrays durch Identifizieren einer Richtung eines Blutflusses bestimmt wird.

6. Elektronische Vorrichtung (601) nach einem der vorhergehenden Ansprüche, wobei die Vielzahl von Sensoren in einer kreuzförmigen Anordnung oder in einer linearen Anordnung ausgelegt sind.

7. Elektronische Vorrichtung (601) nach einem der vorhergehenden Ansprüche, wobei die Empfindlichkeit des mindestens einen Sensors durch Steuern der Spannung des Signals, das dem mindestens einen Sensor bereitgestellt wird, angepasst wird.

8. Elektronische Vorrichtung (601) nach einem der Ansprüche 1 bis 7, die eine transparente Kapselungsschicht (209) umfasst, die auf einer ersten Seite des Erfassungsarrays derart positioniert ist, dass die transparente Kapselungsschicht im Gebrauch zwischen dem Erfassungsarray und einem Patienten positioniert ist.

9. Elektronische Vorrichtung nach [Anspruch] 8, die eine opake Kapselungsschicht (211) umfasst, die mindestens teilweise um einen Rand der transparenten Kapselungsschicht positioniert ist.

10. Verfahren zum Überwachen von mindestens einem biometrischen Parameter, das Folgendes umfasst:
Bereitstellen eines Lichtsignals in einer Richtung zu einem Patienten (301);
Detektieren von Licht, das vom Patienten reflektiert wird, an einem Erfassungsarray (201), wobei das Erfassungsarray eine Vielzahl von Lichtsensoren (203) umfasst, die dazu ausgelegt sind, es zu ermöglichen, dass mindestens ein biometrischer Parameter des Patienten überwacht wird;
Detektieren, dass eine Änderung einer Lichtstärke, die von mindestens einem Sensor im Erfassungsarray detektiert wird, innerhalb eines Schwellwertbereichs liegt; und **dadurch gekennzeichnet, dass** sie Folgendes umfasst:
Bereitstellen eines Ausgangssignals zum Ermöglichen des Anpassens des Erfassungsarrays (201), in Reaktion auf das Detektieren, dass die Änderung innerhalb eines Schwellwertbereichs liegt, wobei das Ausgangssignal bewirkt, dass eine Empfindlichkeit von mindestens einem der Vielzahl von Lichtsensoren (203) angepasst wird, um die detektierte Änderung auszugleichen.

11. Verfahren nach Anspruch 10, wobei das Ausgangssignal bewirkt, dass die Intensität der Lichtquelle angepasst wird, um die detektierte Änderung auszugleichen.

12. Verfahren nach Anspruch 10 oder Anspruch 11, das das Detektieren, dass eine Änderung des Lichts, die von mindestens einem Sensor im Erfassungsarray detektiert wird, innerhalb eines zweiten, anderen Schwellwertbereichs liegt, und in Reaktion auf das Detektieren, dass die Änderung innerhalb des zweiten Schwellwertbereichs liegt, das Bewirken des Bereitstellens einer Anzeige für einen Benutzer, um den Benutzer anzuweisen, die Position des Erfassungsarrays anzupassen, um die detektierte Änderung auszugleichen, umfasst.

13. Verfahren nach Anspruch 12, das das Bestimmen einer Ausrichtung des Erfassungsarrays, um es zu ermöglichen zu bestimmen, wie die Position des Erfassungsarrays angepasst werden sollte, wenn die detektierte Änderung innerhalb des zweiten Schwellwertbereichs liegt, umfasst.

14. Verfahren nach Anspruch 13, wobei die Ausrichtung des Erfassungsarrays durch Identifizieren einer Richtung eines Blutflusses bestimmt wird.

15. Computerprogramm (109), das Computerprogrammanweisungen umfasst, die, wenn sie von einer Verarbeitungsschaltung (105) einer elektronischen Vorrichtung gemäß einem der Ansprüche 1 bis 9 ausgeführt werden, Folgendes bewirken:
Bereitstellen eines Lichtsignals in einer Richtung zum Patienten (301);
Detektieren von Licht, das vom Patienten reflektiert wird, am Erfassungsarray (201), wobei das Erfassungsarray eine Vielzahl von Lichtsensoren (203) umfasst, die dazu ausgelegt sind, es zu ermöglichen, dass mindestens ein biometrischer Parameter des Patienten überwacht wird;
Detektieren, dass eine Änderung einer Lichtstärke, die von mindestens einem Sensor im Erfassungsarray detektiert wird, innerhalb eines Schwellwertbereichs liegt; und **gekennzeichnet durch**
Bereitstellen eines Ausgangssignals zum Ermöglichen des Anpassens des Erfassungsarrays (201) in Reaktion auf das Detektieren, dass die Änderung innerhalb eines Schwellwertbereichs liegt, wobei das Ausgangssignal bewirkt, dass eine Empfindlichkeit von mindestens einem der Vielzahl von Lichtsensoren angepasst wird, um die detektierte Änderung auszugleichen.

## Revendications

1. Dispositif électronique (601) pour surveiller au moins un paramètre biométrique comprenant :
une source de lumière (205) pour fournir un signal lumineux dans une direction vers un sujet (301) ;
un réseau de détection (201) pour détecter une lumière réfléchie par le sujet, dans lequel le réseau de détection comprend une pluralité de capteurs de lumière (203) configurés pour permettre de surveiller au moins un paramètre biométrique du sujet ;
des moyens pour détecter qu'un changement du niveau de lumière détecté par au moins un capteur dans le réseau de détection se situe dans une plage de seuil ; et **caractérisé en ce qu'**il comprend :
des moyens pour fournir, en réponse à la détection du fait que le changement se situe dans une plage de seuil, un signal de sortie permettant de régler le réseau de détection (201), dans lequel le signal de sortie provoque le réglage d'une sensibilité d'au moins un de la pluralité de capteurs de lumière (203) pour compenser le changement détecté.

2. Dispositif électronique (601) selon la revendication 1, dans lequel le signal de sortie provoque le réglage de l'intensité de la source de lumière pour compenser le changement détecté.

3. Dispositif électronique (601) selon l'une quelconque des revendications précédentes comprenant des moyens configurés pour détecter qu'un changement de lumière détecté par au moins un capteur dans le réseau de détection se situe dans une deuxième plage de seuil différente et pour faire en sorte, en réponse à la détection du fait que le changement se situe dans la deuxième plage de seuil, qu'une indication soit fournie à un utilisateur pour demander à l'utilisateur de régler la position du réseau de détection pour compenser le changement détecté.

4. Dispositif électronique (601) selon la revendication 3, comprenant des moyens configurés pour déterminer une orientation du réseau de détection afin de permettre la détermination de la manière dont la position du réseau de détection doit être réglée si le changement détecté se situe dans la deuxième plage de seuil.

5. Dispositif électronique (601) selon la revendication 4, dans lequel l'orientation du réseau de détection est déterminée en identifiant une direction de flux sanguin.

6. Dispositif électronique (601) selon l'une quelconque des revendications précédentes, dans lequel la pluralité de capteurs sont configurés selon un agencement en forme de croix ou selon un agencement linéaire.

7. Dispositif électronique (601) selon l'une quelconque des revendications précédentes, dans lequel la sensibilité du au moins un capteur est réglée en commandant la tension du signal fourni au au moins un capteur.

8. Dispositif électronique (601) selon l'une quelconque des revendications 1 à 7, comprenant une couche d'encapsulation transparente (209) positionnée sur un premier côté du réseau de détection, de sorte qu'en cours d'utilisation, la couche d'encapsulation transparente soit positionnée entre le réseau de détection et un sujet.

9. Dispositif électronique selon [la revendication] 8, comprenant une couche d'encapsulation opaque (211) positionnée au moins partiellement autour d'un bord de la couche d'encapsulation transparente.

10. Procédé pour surveiller au moins un paramètre biométrique comprenant :
la fourniture d'un signal lumineux dans une direction vers un sujet (301) ;
la détection d'une lumière réfléchie par le sujet au niveau d'un réseau de détection (201), dans lequel le réseau de détection comprend une pluralité de capteurs de lumière (203) configurés pour permettre de surveiller au moins un paramètre biométrique du sujet ;
la détection du fait qu'un changement du niveau de lumière détecté par au moins un capteur dans le réseau de détection se situe dans une plage de seuil ; et **caractérisé en ce qu'**il comprend :
la fourniture, en réponse à la détection du fait que le changement se situe dans une plage de seuil, d'un signal de sortie permettant de régler le réseau de détection (201), dans lequel le signal de sortie provoque le réglage d'une sensibilité d'au moins un de la pluralité de capteurs de lumière (203) pour compenser le changement détecté.

11. Procédé selon la revendication 10, dans lequel le signal de sortie provoque le réglage de l'intensité de la source de lumière pour compenser le changement détecté.

12. Procédé selon la revendication 10 ou la revendication 11, comprenant la détection du fait qu'un changement de lumière détecté par au moins un capteur dans le réseau de détection se situe dans une deuxième plage de seuil différente et fait en sorte, en réponse à la détection du fait que le changement se situe dans la deuxième plage de seuil, qu'une indication soit fournie à un utilisateur pour demander à l'utilisateur de régler la position du réseau de détection pour compenser le changement détecté.

13. Procédé selon la revendication 12, comprenant la détermination d'une orientation du réseau de détection afin de permettre la détermination de la manière dont la position du réseau de détection doit être réglée si le changement détecté se situe dans la deuxième plage de seuil.

14. Procédé selon la revendication 13, dans lequel l'orientation du réseau de détection est déterminée en identifiant une direction de flux sanguin.

15. Programme informatique (109) comprenant des instructions de programme informatique qui, lorsqu'elles sont exécutées par un ensemble de circuits de traitement (105) d'un dispositif électronique selon l'une quelconque des revendications 1 à 9, provoquent :
la fourniture d'un signal lumineux dans une direction vers le sujet (301) ;
la détection d'une lumière réfléchie par le sujet au niveau du réseau de détection (201), dans lequel le réseau de détection comprend une pluralité de capteurs de lumière (203) configurés pour permettre de surveiller au moins un paramètre biométrique du sujet ;
la détection du fait qu'un changement du niveau de lumière détecté par au moins un capteur dans le réseau de détection se situe dans une plage de seuil ; et **caractérisé par**
la fourniture, en réponse à la détection du fait que le changement se situe dans une plage de seuil, d'un signal de sortie permettant de régler le réseau de détection (201), dans lequel le signal de sortie provoque le réglage d'une sensibilité d'au moins un de la pluralité de capteurs de lumière pour compenser le changement détecté.
